(19) Europäisches Patentamt
European Patent Office
Office européen des brevets

(11) **EP 3 252 030 B1**

(12) **EUROPEAN PATENT SPECIFICATION**

(45) Date of publication and mention
of the grant of the patent:
**17.10.2018 Bulletin 2018/42**

(51) Int Cl.:
**C07C 7/148** (2006.01)     **C07C 15/073** (2006.01)
**C07C 9/15** (2006.01)

(21) Application number: **16172039.6**

(22) Date of filing: **30.05.2016**

(54) **PROCESS FOR PURIFICATION OF HYDROCARBONS**

VERFAHREN ZUR REINIGUNG VON KOHLENWASSERSTOFFEN

PROCÉDÉ DE PURIFICATION D'HYDROCARBURES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**06.12.2017 Bulletin 2017/49**

(73) Proprietor: **SCG Chemicals Co., Ltd.
Bangkok 10800 (TH)**

(72) Inventors:
• **KETCONG, Anawat
Bangkok 10240 (TH)**
• **KAMMAFOO, Alisa
Rayong 1150 (TH)**

(74) Representative: **Kador & Partner PartG mbB
Corneliusstraße 15
80469 München (DE)**

(56) References cited:
**WO-A1-2015/144778     US-B2- 8 771 501**

## Description

### Field of Invention

The present invention relates to a process for purification of a hydrocarbon mixture using a mixed metal oxides adsorbent.

### Background of Invention

Ethylbenzene is a hydrocarbon compound with high commercial utilization and value. It is majorly used to produce styrene which is an intermediate for polystyrene production. Ethylbenzene can be obtained from alkylation reaction between benzene and ethylene. An alternative way for producing ethylbenzene is to recover it from a hydrocarbon mixture containing ethylbenzene which is generally produced as a by-product stream from several petrochemical processes. However, ethylbenzene directly recovered from a hydrocarbon mixture is typically inferior to that obtained from alkylation reaction in term of purity which may adversely affect further use of ethylbenzene, especially in catalytic conversion processes. One possible source of impurities found in the recovered ethylbenzene stream is the organic solvent contacted with ethylbenzene in the extractive distillation process performed on the hydrocarbon mixture to recover the ethylbenzene. Various organic solvents were disclosed to be capable of extractive separation of ethylbenzene from a hydrocarbon mixture, including chlorinated aromatic compounds.

Methods for selective separation of halogenated compounds from a mixture have been disclosed. For example, US 8,771,501 B2 discloses a process for elimination of chlorine compounds from hydrocarbon cuts involving contacting the hydrocarbon cuts with a first mass comprising palladium on alumina and a second mass comprising alumina promoted with alkali metal or alkaline earth metal. The process needs to be carried out in the presence of hydrogen and at a relatively high temperature.

Another method is disclosed in EP 2017317 B1 which involves selective adsorption of halogenated aromatic compounds using a composition comprising a modified cyclodextrin compound fixed on a solid carrier. The method was used for purification of insulating oils, heat media, lubricating oils, plasticizers, paints, and inks. The method was found to require long contacting time to achieve a good level of purification.

It is an object of the present invention to provide a process for effectively separating impurities, in particular halogenated organic compounds, from a hydrocarbon mixture stream that can overcome drawbacks of the prior art described above.

The present invention provides a process for hydrocarbon purification comprising contacting a hydrocarbon mixture with a mixed metal oxides adsorbent wherein the mixed metal oxides adsorbent comprises, or consists of:

a) an oxide of a first metal which is selected from a metal in oxidation state +1, a metal in oxidation state +2, and mixtures thereof; and

b) an oxide of a second metal which is selected from a metal in oxidation state +3, a metal in oxidation state of +4, and mixtures thereof.

The inventive process is efficient in purification of a hydrocarbon mixture by reducing concentration of impurities, in particular halogenated organic compounds, in the hydrocarbon mixture.

### Brief Description of Drawings

Figure 1 shows results of 1,2,4-trichlorobenzene concentration in effluent streams from the ethylbenzene purification processes employing various adsorbents.

Figure 2 shows results of 1,2,4-trichlorobenzene concentration in effluent streams from the hexane purification processes employing various adsorbents.

### Detailed Description of Invention

The term "oxidation state" is used as defined by IUPAC.

In a preferred embodiment, the atomic ratio of the first metal to the second metal in the mixed metal oxide is in the range of from 0.5:1 to 10:1, more preferably is in the range of from 1:1 to 6:1, and still more preferably is in the range of from 2:1 to 5:1.

Preferably, the first metal is selected from Li, Na, K, Rb, Cs, Fr, Mg, Ca, Mn, Fe, Co, Ni, Cu, Zn, Cd, and mixtures thereof, more preferably from Mg, Ca, Fe, Co, Ni, Cu, and mixtures thereof, even more preferably from Mg, Fe, Ni, and mixtures thereof, and most preferably from Mg, a mixture of Mg and Fe, and a mixture of Mg and Ni.

Also preferably, the second metal is selected from Y, La, Ti, Zr, V, Cr, Mn, Fe, Co, Ni, Cu, Al, Ga, In, Sn, and mixtures thereof, more preferably from Fe, Co, Ni, Cu, Al, Ga, and mixtures thereof, even more preferably from Al, and a mixture of Fe and Al.

At least one of the first metal or the second metal preferably comprises a transition metal selected from the group of Fe, Co, Ni, Cu, and mixtures thereof. The transition metal selected from said group may be present in the mixed metal oxides adsorbent in any of oxidations states +1, +2, +3, and/or +4.

In an embodiment where a transition metal selected from said group is present in the mixed metal oxides adsorbent in oxidation state +1 and/or +2, the transition metal is counted as a part of the first metal. In this embodiment, the atomic ratio of the transition metal selected from said group to the rest of the first metal is preferably in the range of 0.001:1 to 1:1, more preferably 0.01:1 to 0.5:1, and most preferably 0.02:1 to 0.3:1.

In another embodiment, which may or may not be combined with the embodiment described in the preceding paragraph, where a transition metal selected from this group is present in the mixed metal oxides adsorbent in oxidation state +3 and/or +4, the transition metal is counted as a part of the second metal. In this embodiment, the atomic ratio of the transition metal selected from this group to the rest of the second metal is preferably in the range of 0.01:1 to 10:1, more preferably 0.1:1 to 8:1, and most preferably 0.2:1 to 6:1.

The mixed metal oxides adsorbent of the present invention can be prepared by mixing all precursors of the first metal and the second metal together followed by a thermal treatment. "Precursor" refers to any starting compound containing the desired metal which can be converted to its oxide form by a suitable thermal treatment condition. Mixing of precursors can occur in dry form or wet form. When they are mixed in dry form, the precursors may conveniently be provided as powder. Powder of the precursors can be easily mixed by physical mixing in a blender. When they are mixed in wet form, the precursors can be provided as solution and/or suspension. The obtained mixture is subsequently subjected to a thermal treatment. Alternatively, the precursors may be provided in both dry and wet forms. The dry and wet precursors can be combined by any conventional method of heterogeneous adsorbent preparation without limitation including impregnation, incipient wetness, ionexchange, or other methods know in the art. The obtained mixture is then subjected to a thermal treatment to convert the precursors to the oxide form of the first metal and the second metal.

In a preferred embodiment, a layered double hydroxide is used as a precursor to both of the first and the second metals and therefore the mixed metal oxides adsorbent is obtained by subjecting a layered double hydroxide comprising the first metal and the second metal to a thermal treatment.

Layered double hydroxide, also known as LDH, refers to a class of layered material which structurally consists of positively charged mixed metal hydroxide layers and intercalated charge-balancing anions and water between the layers of the structure.

The layered double hydroxide to be used as the precursor to the mixed metal oxides adsorbent according to the present invention appropriately comprises the first metal and the second metal in the amount desired in the resulting mixed metal oxides adsorbent as described above.

The layered double hydroxide can be of natural occurrence or synthesized. In one alternative embodiment, the layered double hydroxide is modified by a modification method prior to subjecting it to the thermal treatment, wherein the modification method comprises contacting a water-wet layered double hydroxide with at least one solvent, the solvent being miscible with water and preferably having a solvent polarity in the range of 3.8 to 9. The layered double hydroxide modification method is described in detail in the patent publication number US2015/0238927A1 and WO2015/144778A1. The modified layered double hydroxide resulting from this modification method has an increased surface area by 34 to 11,000 % and an increased pore volume by 11 to 150,000 % compared to the original layered double hydroxide.

Preferably, for this invention the layered double hydroxide to be used as the precursor to the mixed metal oxides adsorbent has a specific surface area in the range of 100 to 600 $m^2$/g, and more preferably 150 to 500 $m^2$/g.

Thermal treatment refers to any treatment involving subjecting the precursor to a condition and atmosphere that is capable of converting at least a part of the selected precursor to the desired mixed metal oxides adsorbent.

In one specific embodiment, the mixed metal oxides adsorbent is obtained by subjecting a layered double hydroxide to thermal treatment wherein the thermal treatment comprises contacting the layered double hydroxide with a temperature in the range of 100 to 600°C, preferably 350 to 550°C.

Preferably, the thermal treatment is carried out under an atmosphere comprising, or consisting of, a gas selected from an inert gas, an oxidizing gas, and a reducing gas, more preferably selected from nitrogen, hydrogen, and oxygen. The thermal treatment may also preferably be carried out under a atmosphere which comprises, or consists of, a mixture of different gases , such as, for example, a mixture comprising or consisting of, nitrogen and oxygen, such as air. Duration of the thermal treatment is not limited and usually varies with temperature used, but is normally within the range of 1 to 48 hours, more preferably 2 to 30 hours.

In a preferred embodiment, the mixed metal oxides adsorbent of the invention has a pore volume in the range of 0.1 to 3 $cm^3/g$, more preferably 0.3 to 2 $cm^3/g$ still more preferably 0.4 to 1.8 $cm^3/g$, and most preferably 0.5 to 1.6 $cm^3/g$.

Also preferred is the mixed metal oxides adsorbent of the invention has a surface area in the range of 50 to 600 $m^2/g$, more preferably 100 to 500 $m^2/g$.

Furthermore, preferably the mixed metal oxides adsorbent of the invention is in powder form. Still further, preferably it has a particles size of from 20 to 500 microns, more preferably of from 50 to 300 microns. The particle size of the adsorbent is determined by sieving and can be controlled by this method or by any other particle size controlling methods available in the art.

The mixed metal oxides adsorbent described above works efficiently in powder form and, therefore, in a preferred embodiment it is in powder form. It may, however, be formed into different shape and size in order to be more appropriate to mode of operation selected. For example, the mixed metal oxide adsorbent may be suitably formed into pellet, sphere, or extrudate shape for low pressure drop fixed-bed operation mode. In a specific embodiment, the mixed metal oxide is an extrudate form with diameter in the range of 0.1 to 10 mm, more preferably 0.1 to 5 mm.

The mixed metal oxides adsorbent used in the process of the invention is capable of selectively adsorbing impurities, in particular halogenated organic compounds, contained in a hydrocarbon mixture. Therefore, it is effectively employed in a hydrocarbon purification process comprising contacting a hydrocarbon mixture which preferably comprises halogenated organic compounds, with the mixed metal oxides adsorbent.

The term "adsorbing" refers to any kind of interaction that results in binding, physically or chemically, the impurities, in particular halogenated organic compounds, to the adsorbent. The interaction may be reversible or irreversible. Usually, the halogenated organic compound is physically and reversibly bound to the adsorbent.

Prior to contacting with the hydrocarbon mixture, the mixed metal oxides adsorbent may be pretreated to make it ready for adsorption. The pretreatment should at least lead to removal of moisture and/or other substances which might interfere with the function of the adsorbent in the hydrocarbon purification process. Generally, the pretreatment involves contacting the mixed metal oxides adsorbent with a gas at an elevated temperature. Preferably for this invention, the pretreatment comprises treating the mixed metal oxides adsorbent in an inert or oxidizing gas atmosphere, preferably in an atmosphere comprising, or consisting of, nitrogen, a mixture of nitrogen and oxygen, a mixture of nitrogen and air, or air. Preferably, the temperature in the pretreatment step is in the range of 100 to 600°C, more preferably 200 to 500°C, for a period of 1 to 48 hours, more preferably 2 to 12 hours.

The term "hydrocarbon mixture" refers to any hydrocarbon stream, preferably comprising or consisting of hydrocarbons containing 5 to 12 carbon atoms, more preferably 6 to 10 carbon atoms. In one preferred embodiment, the hydrocarbon mixture comprises or consists of aromatic hydrocarbons containing 6 to 10 carbon atoms such as benzene, toluene, ethylbenzene, xylene, and mixtures thereof.

The hydrocarbon mixture to be purified comprises impurities, and preferably comprises one or more halogenated organic compounds as impurities. The term "halogenated organic compound" refers to any organic compound containing at least one heteroatom selected from fluorine, chlorine, bromine, iodine, and astatine. In an embodiment, the halogenated organic compound is an aromatic compound substituted by one or more halogen atoms, preferably chlorine. Examples of the halogenated organic compound contained in the hydrocarbon mixture according to this invention include, but not limited to, polychlorobenzene, polychlorotoluene, and mixtures thereof. More specifically, the impurities contained in the hydrocarbon mixture to be purified comprise, or consist of, one or a mixture of the following compounds: 1,2,4-trichlorobenzene, fluorobenzene, bromobenzene, 4-chlorophenol, fluorene, 1-bromobutane, trichloroethane, and 1,2-dichloro-4-nitrobenzene.

The total amount of impurities, preferably halogenated organic compounds, contained in the hydrocarbon mixture is not limited, but usually the impurities, preferably the halogenated organic compounds, are present in a low concentration only. Typically, the hydrocarbon mixture comprises less than 10 percent by weight, preferably less than 5 percent by weight, and more preferably less than 1 percent by weight, of impurities, preferably halogenated organic compounds.

It was found that adsorption of impurities, in particular halogenated organic compounds, by the mixed metal oxides adsorbent can take place even when no extra energy is supplied to the system. Preferably, contacting the hydrocarbon mixture to be purified with the mixed metal oxide adsorbent is carried out at a temperature in the range of 20 to 150°C, more preferably 20 to 80°C.

Contacting the hydrocarbon mixture to be purified with the mixed metal oxides adsorbent may be carried out in various manners without limitation. Both batch and continuous processes can be used. Typically, continuous processes are more suitable for industrial applications. More particularly, a fixed-bed operational mode, especially with upward flow direction of the hydrocarbon mixture, is preferred.

LHSV is Liquid Hourly Space Velocity. It is a value relating the reactant liquid flow rate to the weight of the adsorbent in the reactor. It can be calculated by the following equation.

$$LHSV\ (h^{-1}) = \frac{Feed\ flow\ rate\ \left(\frac{mL}{min}\right) \times Bulk\ density\ of\ adsorbent\ \left(\frac{g}{mL}\right) \times 60\left(\frac{min}{h}\right)}{Weight\ of\ adsorbent\ (g)}$$

Preferably, contacting the mixed metal oxides adsorbent with the hydrocarbon mixture is carried out in a fixed-bed at operating conditions which allow the hydrocarbon mixture to be present in the liquid phase. The LHSV is preferably in the range of 0.01 to 15 $h^{-1}$, more preferably 0.1 to 10 $h^{-1}$, even more preferably 0.5 to 5 $h^{-1}$.

After having been contacted with the hydrocarbon mixture, there can be hydrocarbon buildups and/or coke deposited on the surface of the mixed metal oxides adsorbent. Deactivation of the adsorbent can be observed from declining adsorption efficiency of the process. Advantageously, the deactivated adsorbent can be regenerated by known techniques for adsorbent regeneration in order to resume its efficiency. Typically, the regeneration procedure involves treating the deactivated adsorbent at an elevated temperature, preferably in an oxidizing atmosphere, to remove at least a portion of hydrocarbon buildups and coke. Preferably, the oxidizing atmosphere comprises, or consists of, oxygen or a gas mixture comprising, or consisting of, oxygen and nitrogen, such as air.

Preferably, in the process of the invention, the mixed metal oxide adsorbent as described herein makes up at least 20 weight% of the total adsorbent used, more preferably makes up at least 50 weight% of the total adsorbent used, still more preferably makes up at least 80 weight% of the total adsorbent used, still more preferably makes up at least 90 weight% of the total adsorbent used, and most preferably the total adsorbent used in the process consists of the mixed metal oxide.

**Examples**

The following examples are intended to be illustrative of this invention only. They are not to be taken in any way limiting on the scope of this invention. Numerous changes and modifications can be made without departing from the scope of the invention as disclosed in the accompanying claims.

Methods for measurement of specific surface area and pore volume:

BET specific surface areas and pore volumes were measured from the $N_2$ adsorption and desorption isotherms at 77 K collected from a Quantachrome Autosorb-6B surface area and pore size analyzer. Before each measurement, LDH samples were first degassed overnight at 110°C.

Method for determining the particle size:

The particles size where mentioned herein was determined by sieving.

Example 1

An adsorbent was prepared by the following steps: 15.3 grams of $Ni(NO_3)_2 \cdot 6H_2O$, 121.2 grams of $Mg(NO_3)_2 \cdot 6H_2O$, and 65.6 grams of $Al(NO_3)_3 \cdot 9H_2O$ were dissolved in 700 mL of deionized water and then mixed with a base solution containing 37.1 grams of $Na_2CO_3$ in 700 mL of deionized water under nitrogen atmosphere. The pH of the solution mixture was controlled to be at 10 by addition of NaOH. After that, the solution mixture was aged for 16 hours at room temperature. The layered double hydroxide precipitated from the solution mixture was filtered out and washed by water until pH is equal to 7. The wet-layered double hydroxide was then washed and dispersed in acetone for 1 hour, filtered, and then dried at 65°C in an oven overnight. The obtained layered double hydroxide was sieved to a particle size of 50 to 300 micrometers. Then the layered double hydroxide was calcined in air at temperature 500°C for 24 hours. The obtained adsorbent contained oxides of Ni (oxidation state +2), Mg (oxidation state +2), and Al (oxidation state +3) wherein an atomic ratio of Ni:Mg:Al was 0.3:2.7:1. The obtained adsorbent furthermore had a surface area of 220 $m^2/g$ and a pore volume of 1.21 $cm^3/g$.

5 grams of the adsorbent was packed in a tube reactor with internal diameter of ¾ inches (1.9 x$10^{-2}$ m) and then pretreated by flowing nitrogen gas through the adsorbent bed at a temperature 400°C for 8 hours.

After the adsorbent bed was cooled down to room temperature and ambient pressure, an adsorption test was performed by feeding a hydrocarbon mixture containing approximately 20 ppm by weight of 1,2,4-trichlorobenzene in ethylbenzene through the adsorbent bed at LHSV 0.8 $h^{-1}$. Effluent from the reactor, collected every 30 minutes or 1 hour, were analyzed by Gas Chromatography (GC) to check remaining concentration of 1,2,4-trichlorobenzene.

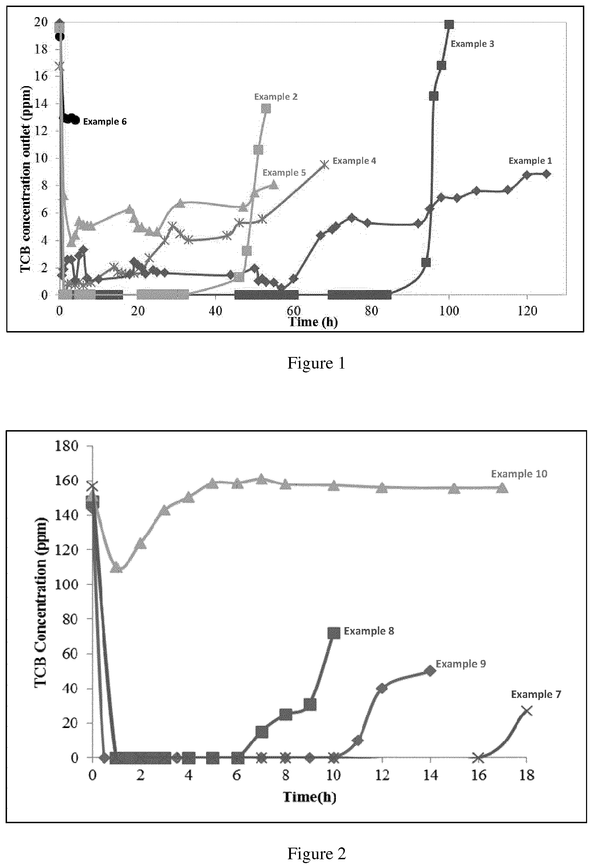

Result from this experiment is shown in Figure 1.

Example 2

An adsorbent was prepared by the following steps: 179.5 grams of $Mg(NO_3)_2 \cdot 6H_2O$, 60.1 grams of $Fe(NO_3)_3 \cdot 9H_2O$, and 9.8 grams of $Al(NO_3)_3 \cdot 9H_2O$ were dissolved in 700 mL of deionized water and then mixed with a base solution containing 37.1 grams of $Na_2CO_3$ in 700 mL of deionized water under nitrogen atmosphere. The pH of the solution mixture was controlled to be at 10 by addition of NaOH. After that, the solution mixture was aged for 2 hours at room temperature and then for 10 hours at hydrothermal conditions in autoclave at 110°C. The layered double hydroxide precipitated from the solution mixture was filtered out and washed by water until pH is equal to 7. The wet-layered double hydroxide was then washed and dispersed in acetone for 1 hour, filtered, and then dried at 65°C in an oven overnight. The obtained layered double hydroxide was sieved to a particle size of 50 to 300 micrometers. Then the layered double hydroxide was calcined in air at temperature 500°C for 24 hours. The obtained adsorbent contained oxides of Mg (oxidation state +2), Fe (oxidation state +3), and Al (oxidation state +3) wherein atomic ratio of Mg:Fe:Al was 4:0.85:0.15. The obtained adsorbent furthermore had a surface area of 220 m$^2$/g and a pore volume of 1.27 cm$^3$/g.

The obtained adsorbent was pretreated and subjected to an adsorption test as described in Example 1. Result from this experiment is shown in Figure 1.

Example 3

An adsorbent was prepared by the following steps: 179.5 grams of Mg(NO$_3$)$_2$·6H$_2$O, 24.7 grams of Fe(NO$_3$)$_3$·9H$_2$O, and 42.7 grams of Al(NO$_3$)$_3$·9H$_2$O were dissolved in 700 mL of deionized water and then mixed with a base solution containing 37.1 grams of Na$_2$CO$_3$ in 700 mL of deionized water under nitrogen atmosphere. The pH of the solution mixture was controlled to be at 10 by addition of NaOH. After that, the solution mixture was aged for 2 hours at room temperature and then for 10 hours at hydrothermal conditions in autoclave at 110°C. The layered double hydroxide precipitated from the solution mixture was filtered out and washed by water until pH is equal to 7. The wet-layered double hydroxide was then washed and dispersed in acetone for 1 hour, filtered, and then dried at 65°C in an oven overnight. The obtained layered double hydroxide was sieved to a particle size of 50 to 300 micrometers. Then the layered double hydroxide was calcined in air at temperature 500°C for 8 hours. The obtained adsorbent contained oxides of Mg (oxidation state +2), Fe (oxidation state +3), and Al (oxidation state +3) wherein atomic ratio of Mg:Fe:Al was 4:0.35:0.65. The obtained adsorbent furthermore had a surface area of 240 m$^2$/g and a pore volume of 1.68 cm$^3$/g.

The obtained adsorbent was pretreated and subjected to an adsorption test as described in Example 1. Result from this experiment is shown in Figure 1.

Example 4

An adsorbent was prepared by the following steps: 179.5 grams of Mg(NO$_3$)$_2$·6H$_2$O, and 65.6 grams of Al(NO$_3$)$_3$·9H$_2$O were dissolved in 700 mL of deionized water and then mixed with a base solution containing 37.1 grams of Na$_2$CO$_3$ in 700 mL of deionized water under nitrogen atmosphere. The pH of the solution mixture was controlled to be at 10 by addition of NaOH. After that, the solution mixture was aged for 16 hours at room temperature. The layered double hydroxide precipitated from the solution mixture was filtered out and washed by water until pH is equal to 7. The wet-layered double hydroxide was then washed and dispersed in acetone for 1 hour, filtered, and then dried at 65°C in an oven overnight. The obtained layered double hydroxide was sieved to a particle size of approximately 1-2 millimeters. Then the layered double hydroxide was calcined in air at temperature 500°C for 24 hours. The obtained adsorbent contained oxides of Mg (oxidation state +2) and Al (oxidation state +3) wherein an atomic ratio of Mg:Al was 4:1. The obtained adsorbent furthermore had a surface area of 140 m$^2$/g and a pore volume of 0.64 cm$^3$/g.

The obtained adsorbent was pretreated and subjected to an adsorption test as described in Example 1. Result from this experiment is shown in Figure 1.

Example 5

KW-2000, which is a commercially available calcined hydrotalcite (LDH comprising 3Mg:1Al and CO$_3$ anions), calcined at 500°C for 24 hours) from Kyowa Chemical Industry Co., Ltd., Japan, was used as an adsorbent. The adsorbent had a surface area of 190 m$^2$/g and a pore volume of 1.26 cm$^3$/g. The adsorbent was pretreated and subjected to an adsorption test as described in Example 1. Result from this experiment is shown in Figure 1.

Example 6 (Comparative)

CLR-454, which is a commercially available molecular sieve (SiO$_2$/Al$_2$O$_3$ (molecular sieve) + Al$_2$O$_3$) from UOP, was used as an adsorbent. It had a surface area of 270 m$^2$/g and a pore volume of 0.33 cm$^3$/g. The adsorbent was pretreated and subjected to an adsorption test as described in Example 1. Result from this experiment is shown in Figure 1.Result from this experiment is shown in Figure 1.

It can be seen from the results of the above examples that the inventive process (Example 1, 2, 3, 4, and 5) can effectively reduce concentration of 1,2,4-trichlorobenzene in ethylbenzene and show good adsorption stability well over 40 hours before reaching adsorption breakthrough. In contrast, a process using the conventional adsorbent of Comparative Example 6 allows reduction of the concentration of 1,2,4-trichlorobenzene only to a significantly lower extent for a very short period.

Example 7

An adsorbent was prepared by the steps as described in Example 3.

The adsorbent was pretreated by flowing nitrogen gas through the adsorbent bed at a temperature 400°C for 8 hours. After the adsorbent bed was cooled down to room temperature and ambient pressure, an adsorption test was performed by feeding a hydrocarbon mixture containing approximately 150 ppm by weight of 1,2,4-trichlorobenzene in hexane through the adsorbent bed at LHSV 0.8 h-1. Effluent from the reactor, collected every 30 minutes or 1 hour, were analyzed by Gas Chromatography (GC) to check remaining concentration of 1,2,4-trichlorobenzene.

Result from this experiment is shown in Figure 2.

Example 8 (Comparative)

Magnesium oxide (MgO) was used as an adsorbent. It had a surface area of 32 $m^2/g$ and a pore volume of 0.16 $cm^3/g$. The adsorbent was pretreated and subjected to an adsorption test as described in Example 7. Result from this experiment is shown in Figure 2.

Example 9 (Comparative)

Aluminum dioxide ($Al_2O_3$) was used as an adsorbent. It had a surface area of 300 $m^2/g$ and a pore volume of 0.35 $cm^3/g$. The adsorbent was pretreated by flowing nitrogen gas through the adsorbent bed at a temperature 300°C for 4 hours and then subjected to an adsorption test as described in Example 7. Result from this experiment is shown in Figure 2.

Example 10 (Comparative)

Iron oxide ($Fe_2O_3$) was used as an adsorbent. It had a surface area of 8.7 $m^2/g$ and a pore volume of 0.28 $cm^3/g$. The adsorbent was pretreated by flowing nitrogen gas through the adsorbent bed at a temperature 400°C for 4 hours and then subjected to an adsorption test as described in Example 7. Result from this experiment is shown in Figure 2.

The inventive process using the adsorbent of Example 7 shows better hexane purification efficiency compared to comparative process using the adsorbents of Example 8, 9, and 10.

**Claims**

1.   A process for hydrocarbon purification comprising contacting a hydrocarbon mixture with a mixed metal oxides adsorbent wherein the mixed metal oxides adsorbent comprises:

   a) an oxide of a first metal which is selected from a metal in oxidation state +1, a metal in oxidation state +2, and mixtures thereof; and
   b) an oxide of a second metal which is selected from a metal in oxidation state +3, a metal in oxidation state +4, and mixtures thereof.

2.   The process for hydrocarbon purification according to claim 1 wherein the atomic ratio of the first metal and the second metal is in the range of 0.5:1 to 10:1.

3.   The process for hydrocarbon purification according to claim 1 or 2 wherein the hydrocarbon mixture to be purified comprises halogenated organic compounds.

4.   The process for hydrocarbon purification according to claim 3 wherein the halogenated organic compound is selected from polychlorobenzene, polychlorotoluene, and mixtures thereof.

5.   The process for hydrocarbon purification according to any of the preceding claims wherein the first metal is selected from Li, Na, K, Rb, Cs, Fr, Mg, Ca, Mn, Fe, Co, Ni, Cu, Zn, Cd, and mixtures thereof.

6.   The process for hydrocarbon purification according to any of the preceding claims wherein the second metal is selected from Y, La, Ti, Zr, V, Cr, Mn, Fe, Co, Ni, Cu, Al, Ga, In, Sn, and mixtures thereof.

7. The process for hydrocarbon purification according to any of the preceding claims wherein at least one of the first metal or the second metal comprises a transition metal selected from Fe, Co, Ni, Cu, and mixtures thereof.

8. The process for hydrocarbon purification according to claim 7 wherein the transition metal is in oxidation state +1 and/or +2 and the atomic ratio of the transition metal to the rest of the first metal is in the range of 0.001:1 to 1:1.

9. The process for hydrocarbon purification according to claim 7 or 8 wherein the transition metal is in oxidation state +3 and/or +4 and the atomic ratio of the transition metal to the rest of the second metal is in the range of 0.01:1 to 10:1.

10. The process for hydrocarbon purification according to any of the preceding claims wherein the mixed metal oxides adsorbent has a pore volume in the range of 0.1 to 3 $cm^3/g$.

11. The process for hydrocarbon purification according to any of the preceding claims wherein the mixed metal oxides adsorbent has a particle size of from 20 to 500 microns.

12. The process for hydrocarbon purification according to any of the preceding claims wherein the mixed metal oxides adsorbent is obtained by subjecting a layered double hydroxide comprising the first metal and the second metal to a thermal treatment.

13. The process for hydrocarbon purification according to claim 12 wherein the layered double hydroxide has a specific surface area in the range of 100 to 600 $m^2/g$.

14. The process for hydrocarbon purification according to claim 12 or 13 wherein the thermal treatment comprises contacting the layered double hydroxide with a temperature in the range of 100 to 600°C.

15. The process for hydrocarbon purification according to any of the preceding claims wherein the process further comprises pretreatment of the mixed metal oxides adsorbent comprising contacting the mixed metal oxides adsorbent with an inert gas or oxidizing gas atmosphere at a temperature in the range of 100 to 600°C.

16. The process for hydrocarbon purification according to any of the preceding claims wherein the hydrocarbon mixture comprises a hydrocarbon containing 5 to 12 carbon atoms.

17. The process for hydrocarbon purification according to any of the preceding claims wherein contacting the hydrocarbon mixture with the mixed metal oxide adsorbent is carried out at a temperature in the range of 20 to 150°C.

18. The process for hydrocarbon purification according to any of the preceding claims wherein the process further comprises regenerating the mixed metal oxides adsorbent.

19. Use of a mixed metal oxides adsorbent wherein which comprises:

a) an oxide of a first metal which is selected from a metal in oxidation state +1, a metal in oxidation state +2, and mixtures thereof; and
b) an oxide of a second metal which is selected from a metal in oxidation state +3, a metal in oxidation state +4, and mixtures thereof

for the adsorptive purification of a hydrocarbon mixture.

**Patentansprüche**

1. Verfahren zur Kohlenwasserstoffreinigung, umfassend das In-Kontakt-Bringen von einer Kohlenwasserstoffmischung mit einem gemischte Metalloxide-Adsorptionsmittel, wobei das gemischte Metalloxide-Adsorptionsmittel das Folgende umfasst:

a) ein Oxid eines ersten Metalls, das aus einem Metall im Oxidationszustand +1, einem Metall im Oxidationszustand +2 und Mischungen davon ausgewählt ist; und

b) ein Oxid eines zweiten Metalls, das aus einem Metall im Oxidationszustand +3, einem Metall im Oxidationszustand +4 und Mischungen davon ausgewählt ist.

2. Verfahren zur Kohlenwasserstoffreinigung nach Anspruch 1, wobei das Atomverhältnis von dem ersten Metall und dem zweiten Metall in dem Bereich von 0,5:1 bis 10:1 liegt.

3. Verfahren zur Kohlenwasserstoffreinigung nach Anspruch 1 oder 2, wobei die zu reinigende Kohlenwasserstoffmischung halogenierte organische Verbindungen umfasst.

4. Verfahren zur Kohlenwasserstoffreinigung nach Anspruch 3, wobei die halogenierte organische Verbindung aus Polychlorbenzol, Polychlortoluol und Mischungen davon ausgewählt ist.

5. Verfahren zur Kohlenwasserstoffreinigung nach einem der vorhergehenden Ansprüche, wobei das erste Metall aus Li, Na, K, Rb, Cs, Fr, Mg, Ca, Mn, Fe, Co, Ni, Cu, Zn, Cd und Mischungen davon ausgewählt ist.

6. Verfahren zur Kohlenwasserstoffreinigung nach einem der vorhergehenden Ansprüche, wobei das zweite Metall aus Y, La, Ti, Zr, V, Cr, Mn, Fe, Co, Ni, Cu, Al, Ga, In, Sn und Mischungen davon ausgewählt ist.

7. Verfahren zur Kohlenwasserstoffreinigung nach einem der vorhergehenden Ansprüche, wobei mindestens eines aus dem ersten Metall oder dem zweiten Metall ein Übergangsmetall umfasst, das aus Fe, Cu, Ni, Cu und Mischungen davon ausgewählt ist.

8. Verfahren zur Kohlenwasserstoffreinigung nach Anspruch 7, wobei das Übergangsmetall im Oxidationszustand +1 und/oder +2 vorliegt und das Atomverhältnis von dem Übergangsmetall zu dem Rest des ersten Metalls in dem Bereich von 0,001:1 bis 1:1 liegt.

9. Verfahren zur Kohlenwasserstoffreinigung nach Anspruch 7 oder 8, wobei das Übergangsmetall im Oxidationszustand +3 und/oder +4 vorliegt und das Atomverhältnis von dem Übergangsmetall zu dem Rest des zweiten Metalls in dem Bereich von 0,01:1 bis 10:1 liegt.

10. Verfahren zur Kohlenwasserstoffreinigung nach einem der vorhergehenden Ansprüche, wobei das gemischte Metalloxide-Adsorptionsmittel ein Porenvolumen in dem Bereich von 0,1 bis 3 $cm^3$/g aufweist.

11. Verfahren zur Kohlenwasserstoffreinigung nach einem der vorhergehenden Ansprüche, wobei das gemischte Metalloxide-Adsorptionsmittel eine Partikelgröße von 20 bis 500 Mikrometern aufweist.

12. Verfahren zur Kohlenwasserstoffreinigung nach einem der vorhergehenden Ansprüche, wobei das gemischte Metalloxide-Adsorptionsmittel erhalten wird, indem ein geschichtetes Doppelhydroxid, das das erste Metall und das zweite Metall umfasst, einer Wärmebehandlung unterzogen wird.

13. Verfahren zur Kohlenwasserstoffreinigung nach Anspruch 12, wobei das geschichtete Doppelhydroxid einen spezifischen Oberflächenbereich in dem Bereich von 100 bis 600 $m^2$/g aufweist.

14. Verfahren zur Kohlenwasserstoffreinigung nach Anspruch 12 oder 13, wobei die Wärmebehandlung das In-Kontakt-Bringen von dem geschichteten Doppelhydroxid mit einer Temperatur in dem Bereich von 100 bis 600 °C umfasst.

15. Verfahren zur Kohlenwasserstoffreinigung nach einem der vorhergehenden Ansprüche, wobei das Verfahren ferner eine Vorbehandlung von dem gemischte Metalloxide-Adsorptionsmittel umfasst, die das In-Kontaktgeringen von dem gemischte Metalloxide-Adsorptionsmittel mit einer Inertgas- oder Oxidationsgasatmosphäre bei einer Temperatur in dem Bereich von 100 bis 600 °C umfasst.

16. Verfahren zur Kohlenwasserstoffreinigung nach einem der vorhergehenden Ansprüche, wobei die Kohlenwasserstoffmischung einen Kohlenwasserstoff umfasst, der 5 bis 12 Kohlenstoffatome enthält.

17. Verfahren zur Kohlenwasserstoffreinigung nach einem der vorhergehenden Ansprüche, wobei das In-Kontakt-Bringen von der Kohlenwasserstoffmischung mit dem gemischten Metalloxid-Adsorptionsmittel bei einer Temperatur in dem Bereich von 20 bis 150 °C durchgeführt wird.

**18.** Verfahren zur Kohlenwasserstoffreinigung nach einem der vorhergehenden Ansprüche, wobei das Verfahren ferner das Regenerieren von dem gemischte Metalloxide-Adsorptionsmittel umfasst.

**19.** Verwendung von einem gemischte Metalloxide-Adsorptionsmittel, wobei das gemischte Metalloxide-Adsorptionsmittel das Folgende umfasst:

a) ein Oxid eines ersten Metalls, das aus einem Metall im Oxidationszustand +1, einem Metall im Oxidationszustand +2 und Mischungen davon ausgewählt ist; und
b) ein Oxid eines zweiten Metalls, das aus einem Metall im Oxidationszustand +3, einem Metall im Oxidationszustand +4 und Mischungen davon ausgewählt ist

zur adsorptiven Reinigung von einer Kohlenwasserstoffmischung.

**Revendications**

**1.** Procédé pour la purification d'hydrocarbures, comprenant la mise en contact d'un mélange d'hydrocarbures avec un adsorbant à base d'oxydes métalliques mixtes, dans lequel l'adsorbant à base d'oxydes métalliques mixtes comprend :

a) un oxyde d'un premier métal qui est choisi parmi un métal dans l'état d'oxydation +1, un métal dans l'état d'oxydation +2, et leurs mélanges ; et
b) un oxyde d'un deuxième métal qui est choisi parmi un métal dans l'état d'oxydation +3, un métal dans l'oxydation +4, et leurs mélanges.

**2.** Procédé pour la purification d'hydrocarbures selon la revendication 1, dans lequel le rapport atomique du premier métal au deuxième métal est situé dans la plage allant de 0,5/1 à 10/1.

**3.** Procédé pour la purification d'hydrocarbures selon la revendication 1 ou 2, dans lequel le mélange d'hydrocarbures devant être purifié comprend des composés organiques halogénés.

**4.** Procédé pour la purification d'hydrocarbures selon la revendication 3, dans lequel le composé organique halogéné est choisi parmi le polychlorobenzène, le polychlorotoluène, et leurs mélanges.

**5.** Procédé pour la purification d'hydrocarbures selon l'une quelconque des revendications précédentes, dans lequel le premier métal est choisi parmi Li, Na, K, Rb, Cs, Fr, Mg, Ca, Mn, Fe, Co, Ni, Cu, Zn, Cd, et leurs mélanges.

**6.** Procédé pour la purification d'hydrocarbures selon l'une quelconque des revendications précédentes, dans lequel le deuxième métal est choisi parmi Y, La, Ti, Zr, V, Cr, Mn, Fe, Co, Ni, Cu, Al, Ga, In, Sn, et leurs mélanges.

**7.** Procédé pour la purification d'hydrocarbures selon l'une quelconque des revendications précédentes, dans lequel au moins l'un parmi le premier métal et le deuxième métal comprend un métal de transition choisi parmi Fe, Co, Ni, Cu, et leurs mélanges.

**8.** Procédé pour la purification d'hydrocarbures selon la revendication 7, dans lequel le métal de transition est dans l'état d'oxydation +1 et/ou +2 et le rapport atomique du métal de transition au reste du premier métal est situé dans la plage allant de 0,001/1 à 1/1.

**9.** Procédé pour la purification d'hydrocarbures selon la revendication 7 ou 8, dans lequel le métal de transition est dans l'état d'oxydation +3 et/ou +4 et le rapport atomique du métal de transition au reste du deuxième métal est situé dans la plage allant de 0,01/1 à 10/1.

**10.** Procédé pour la purification d'hydrocarbures selon l'une quelconque des revendications précédentes, dans lequel l'adsorbant à base d'oxydes métalliques mixtes a un volume de pores situé dans la plage allant de 0,1 à 3 $cm^3/g$.

**11.** Procédé pour la purification d'hydrocarbures selon l'une quelconque des revendications précédentes, dans lequel l'adsorbant à base d'oxydes métalliques mixtes a une granulométrie de 20 à 500 micromètres.

**12.** Procédé pour la purification d'hydrocarbures selon l'une quelconque des revendications précédentes, dans lequel l'adsorbant à base d'oxydes métalliques mixtes est obtenu par soumission d'un hydroxyde double stratifié comprenant le premier métal et le deuxième métal à un traitement thermique.

**13.** Procédé pour la purification d'hydrocarbures selon la revendication 12, dans lequel l'hydroxyde double stratifié a une surface spécifique située dans la plage allant de 100 à 600 m$^2$/g.

**14.** Procédé pour la purification d'hydrocarbures selon la revendication 12 ou 13, dans lequel le traitement thermique comprend la mise en contact de l'hydroxyde double stratifié à une température située dans la plage allant de 100 à 600°C.

**15.** Procédé pour la purification d'hydrocarbures selon l'une quelconque des revendications précédentes, lequel procédé comprend en outre un prétraitement de l'adsorbant à base d'oxydes métalliques mixtes, comprenant la mise en contact des oxydes métalliques mixtes avec une atmosphère de gaz inerte ou de gaz oxydant à une température située dans la plage allant de 100 à 600°C.

**16.** Procédé pour la purification d'hydrocarbures selon l'une quelconque des revendications précédentes, dans lequel le mélange d'hydrocarbures comprend un hydrocarbure contenant 5 à 12 atomes de carbone.

**17.** Procédé pour la purification d'hydrocarbures selon l'une quelconque des revendications précédentes, dans lequel la mise en contact du mélange d'hydrocarbures avec l'adsorbant à base d'oxydes métalliques mixtes est effectuée à une température située dans la plage allant de 20 à 150°C.

**18.** Procédé pour la purification d'hydrocarbures selon l'une quelconque des revendications précédentes, lequel procédé comprend en outre la régénération de l'adsorbant à base d'oxydes métalliques mixtes.

**19.** Utilisation d'un adsorbant à base d'oxydes métalliques mixtes qui comprend :

a) un oxyde d'un premier métal qui est choisi parmi un métal dans l'état d'oxydation +1, un métal dans l'état d'oxydation +2, et leurs mélanges ; et
b) un oxyde d'un deuxième métal qui est choisi parmi un métal dans l'état d'oxydation +3, un métal dans l'oxydation +4, et leurs mélanges,

pour la purification par adsorption d'un mélange d'hydrocarbures.

Figure 1

Figure 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8771501 B2
- EP 2017317 B1
- US 20150238927 A1
- WO 2015144778 A1